# FASCICULE DE BREVET EUROPEEN

(11) **EP 0 709 081 B1**
(45) Date de publication et mention de la délivrance du brevet: **25.06.1997**
(21) Numéro de dépôt: 95401986.5
(22) Date de dépôt: 31.08.1995
(51) Int. Cl.: A61K 7/42, A61K 7/48

(54) **Composition contenant un précurseur de la dihydroxyacetone**
Zusammensetzung, die einen Precursor von Dihydroxyaceton enthaelt
Composition containing a precursor of dihydroacetone

(30) Priorité: 24.10.1994 FR 9412686
(43) Date de publication de la demande: 01.05.1996
(73) Titulaire: L'OREAL, 75008 Paris (FR)
(72) Inventeur: Philippe, Michel, F-91320 Wissous (FR); Tuloup, Rémy, F-75014 Paris (FR); de Salvert, Armelle, F-75013 Paris (FR); Sera, Daniel, F-94150 L'Hay-Les-Roses (FR); Fodor, Pierre, F-92380 Garches (FR)
(74) Mandataire: Tezier Herman, Béatrice

(56) Documents cités:
- EP-A- 0 547 864
- WO-A-94/04130
- WO-A-94/13258
- WO-A-94/22419

## Description

L'invention concerne une composition cosmétique ou dermatologique, contenant au moins un précurseur d'une substance dont la forme active est recherchée pour son activité en cosmétique. L'une des applications de l'invention concerne les compositions capables de conférer rapidement à la peau une couleur similaire à celle obtenue lors d'une exposition prolongée aux rayonnements ultraviolets, solaires ou artificiels, tout en évitant les inconvénients d'une telle exposition (érythèmes, brûlures, perte d'élasticité, apparition de rides, vieillissement prématuré de la peau, et autres).

L'art antérieur enseigne depuis de longues années l'implication de la dihydroxyacétone (DHA par la suite dans le texte), dans la coloration artificielle de la peau (Bobin et al. J. Soc. Cosmet. Chem., 35 pages 265-272, 1984). La DHA réagit avec les acides aminés naturellement contenus dans le film lipidique du stratum corneum et par une réaction de Maillard forme des mélanoïdes (Maillard L.C., C.R. Acad. Sci. 154, 66-68, 1912).

Des compositions cosmétiques utilisées dans le but de colorer artificiellement la peau et contenant de la DHA sont largement décrites dans l'art antérieur comme par exemple dans la demande de brevet FR-A-2597345 de la demanderesse.

Afin d'améliorer les effets de la DHA, celle-ci est souvent associée à d'autres substances en vue d'augmenter la rapidité de l'apparition de la couleur ou la résistance de celle-ci dans le temps. On peut citer par exemple les associations décrites dans les demandes WO-A-9404130 ou EP-A-547864.

L'utilisation de la DHA présente de nombreux inconvénients peu compatibles avec l'attrait du consommateur. En effet, la stabilité de la DHA en formulation est tout à fait relative, entraînant au cours du temps une dégradation du composé. On constate en particulier que les compositions contenant de la DHA prennent parfois, avant utilisation, une coloration peu agréable aux yeux des utilisateurs. En outre, au cours du temps il peut apparaître, pour ces mêmes compositions, une odeur nauséabonde, désagréable qui est en général mal perçue par le consommateur. Le pH de la composition diminue également au cours du temps, rendant celle-ci à terme incompatible avec un emploi en application topique.

Par ailleurs, si l'on s'en tient à l'activité de la DHA, il est connu que sa rémanence sur la peau n'est pas parfaite.

C'est afin de résoudre ces inconvénients que la demanderesse propose une nouvelle composition cosmétique dont l'un des buts est de fournir au moment de l'application de celle-ci, en particulier sur la peau, de la DHA, sans pour autant que ladite composition contienne de la DHA en tant que telle.

Ainsi un premier objet de l'invention concerne une composition cosmétique ou dermatologique, comprenant au moins un précurseur de la DHA.

L'invention concerne donc une composition cosmétique ou dermatologique, contenant dans un véhicule cosmétiquement ou dermatologiquement acceptable, au moins un dérivé estérifié de la dihydroxyacétone répondant à la formule générale : 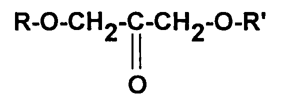 dans laquelle R et R' représentent un atome d'hydrogène ou un radical acyle éventuellement hydroxylé, ayant de 2 à 25 atomes de carbone, linéaire ou ramifié, ou cyclique, saturé ou insaturé, R et R' pouvant être identiques ou différents à la condition qu'ils ne soient jamais simultanément un atome d'hydrogène.

De manière préférentielle R et R' sont un radical benzoyle ou un radical alkylbenzoyle ou un radical acylbenzoyle ou un radical 2-hydroxy-2-phénylacétyle, éventuellement hydroxylé.

L'invention telle que décrite précédemment permet d'améliorer la rémanence du produit appliqué ainsi que sa stabilité en formulation. Il n'en demeure pas moins que l'un des principaux intérêts recherché pour une telle composition cosmétique ou dermatologique, à savoir utiliser la DHA pour ses propriétés, ne peut être atteint que si le dérivé estérifié est transformé au moment de l'application en DHA. Une telle hydrolyse, qui au moment de l'application libérera de la DHA, n'est possible qu'en présence d'un (ou plusieurs) composé(s) capable(s) de couper la (ou les) liaison(s) ester(s) du dérivé. On trouve de tels composés sur la peau. Cependant, afin d'améliorer l'efficacité de la composition selon l'invention, il est souhaitable de fournir le dérivé estérifié de la DHA et le composé capable de couper une liaison ester en même temps.

Ainsi un second objet de l'invention concerne une composition comprenant au moins un dérivé estérifié de la dihydroxyacétone répondant à la formule générale : 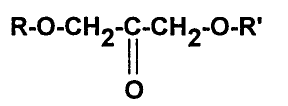 dans laquelle R et R' représentent un atome d'hydrogène ou un radical acyle éventuellement hydroxylé, ayant de 2 à 25 atomes de carbone, linéaire ou ramifié, ou cyclique, saturé ou insaturé, R et R' pouvant être identiques ou différents à la condition qu'ils ne soient jamais simultanément un atome d'hydrogène,
et au moins un composé capable de couper au moins une liaison ester.

De manière préférentielle R et R' sont un radical benzoyle ou un radical alkylbenzoyle ou un radical acylbenzoyle ou un radical 2-hydroxy-2-phénylacétyle, éventuellement hydroxylé.

Avantageusement, cette composition pourra être sous une forme cosmétiquement ou dermatologiquement acceptable, en vue de son utilisation dans les domaines considérés.

Selon un mode préférentiel de réalisation de l'invention le radical acyle a de 3 à 18 atomes de carbone.

De façon avantageuse, le dérivé estérifié peut être choisi dans le groupe constitué du :
- 1,3-didodécanoate de 2-oxo-propane,
- 1,3-dihéxadécanoate de 2-oxo-propane,
- 3-hexadécanoate de 2-oxo-propanol.

Selon un mode préféré de réalisation de l'invention, le dérivé estérifié peut être à une concentration allant de 0,1% à 20% et de préférence à une concentration allant de 0,5% à 10%.
Ici et dans la suite du texte les pourcentages sont donnés en poids par rapport au poids total de la composition.

Selon le deuxième objet de l'invention, et dans un mode de réalisation particulier de celui-ci, le composé capable de couper au moins une liaison ester peut être tout composé nucléophile acceptable en cosmétique. Ainsi on peut citer, les alcools, les thiols, les amines, ou les anions.

Parmi les amines on choisira de préférence une amine hydroxylée, comme par exemple la 3-amino-1,2-propanediol, la 2-amino-2-méthyl-1,3-propanediol, la 2-amino-2-méthyl-propanol, la 2-amino-2-hydroxyméthyl-1,3-propanediol, la glucamine, la n-méthyl-glucamine, ou un acide aminé comme par exemple la lysine, l'arginine ou l'histidine.

Parmi les anions on choisira de préférence un anion carboxylate, comme par exemple les sels d'acides gras, d'amines acides ou de lipoaminoacides.

Les enzymes sont la seconde grande famille de composés capables de couper au moins une liaison ester, utilisables dans l'invention. On peut citer par exemples les hydrolases, parmi lesquelles on citera de manière non limitative, les lipases, les estérases ou les protéases. Dans les lipases on choisira de préférence une lipase de pancréas de porc comme celle vendue sous la dénomination Type II par la société Sigma ou encore de la lipolase SP 644 vendue par la société Novo-Nordisk.

Selon le deuxième objet de l'invention, et dans un autre mode particulier de réalisation de celui-ci, le composé capable de couper au moins une liaison ester peut être utilisé à une concentration allant de 0,1% à 30% et de préférence allant de 0,5% à 15%.

Il est cependant préférable, dans l'usage courant de telles compositions de faire en sorte que l'hydrolyse du dérivé estérifié de la DHA ne s'effectue qu'au moment de l'application de la (ou des) composition(s). Il est donc avantageux de prévoir un conditionnement tel que le dérivé estérifié et le composé capable de couper au moins une liaison ester soient conditionnés de sorte à ne pas être au contact l'un de l'autre.

Un troisième objet de l'invention concerne donc une composition dans laquelle le dérivé estérifié et le composé capable de couper au moins une liaison peuvent être conditionnés de sorte à ne pas être au contact l'un de l'autre.

Les deux compartiments séparés solidaires, peuvent par exemple constituer un conditionnement unique sous forme d'un tube souple, tel que le mélange du dérivé estérifié et du composé capable de couper au moins une liaison ester ne s'effectue que lorsque chacun d'eux est expulsé de son compartiment propre. Cette expulsion peut être simultanée ou non. C'est au cours de l'application que le mélange s'effectuera.

Une autre forme de conditionnement à deux compartiments séparés solidaires peut être telle que le dérivé estérifié ou le composé capable de couper une liaison ester est encapsulé sous forme de microcapsules, de sphérules ou tout autre forme connue de l'homme du métier, et est conditionné en présence de l'autre élément de l'invention sous une forme différente, elle-même connue de l'homme du métier.

Dans une forme cosmétique ou dermatologique de ce conditionnement, la partie non encapsulée de l'invention pourra être une crème, un gel ou tout autre forme connue de l'homme du métier.
Rien ne s'oppose au fait que les deux éléments soient chacun encapsulé. Là encore, l'homme du métier sait préparer de telles formes.
C'est au cours de l'application que s'effectuera la libération, par écrasement des capsules sous la pression de l'utilisateur et le mélange des compositions ainsi libérées.

Quelle que soit la forme de réalisation de l'invention, dans une application cosmétique ou dermatologique, elle peut en outre contenir tout autre constituant cosmétiquement ou dermatologiquement acceptable habituellement utilisé dans ce genre de composition, et en particulier les additifs utilisés pour augmenter l'efficacité de la DHA issue de l'hydrolyse du dérivé.

La composition cosmétique ou dermatologique selon l'une quelconque des formes de réalisation de l'invention et en particulier les compositions distinctes contenant respectivement le dérivé estérifié et le composé peuvent se présenter comme une huile, un gel, une émulsion, une dispersion vésiculaire.

Un quatrième objet de l'invention concerne l'utilisation de ces compositions cosmétiques ou dermatologiques, dans le but de colorer artificiellement la peau.

Un cinquième objet de l'invention concerne un procédé de coloration artificiel de la peau utilisant, en application, une composition telle que décrite précédemment dans le texte.

On va maintenant donner des exemples de compositions selon l'invention.

### Exemple 1 : Crème solaire autobronzante

A. Emulsion contenant l'ester de dihydroxyacétone :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |
| Palmitate de dihydroxyacétone | 14,6 % |

| *Phase aqueuse :* | |
|---|---|
| Phénoxyéthanol (conservateur) | 0,5 % |
| Eau | qsp 100 % |

B. Emulsion contenant la lipase :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |

| *Phase aqueuse :* | |
|---|---|
| Phénoxyéthanol (conservateur) | 0,5 % |
| Lipase SP644 | 2 % |
| Eau | qsp 100 % |

Les émulsions A et B sont disposées dans deux compartiments séparés et mélangées au moment de l'application sur la peau.

Le produit obtenu donne, après application sur la peau, une coloration progressivement bronzée à la peau.

### Exemple 2 : Crème solaire autobronzante

A. Emulsion contenant l'ester de dihydroxyacétone :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |
| Laurate de dihydroxyacétone | 10 % |

| *Phase aqueuse :* | |
|---|---|
| Phénoxyéthanol (conservateur) | 0,5 % |
| Eau | qsp 100 % |

B. Emulsion contenant la lipase :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |

| *Phase aqueuse :* | |
|---|---|
| Phénoxyéthanol (conservateur) | 0,5 % |
| Lipase 100 L | 1 % |
| Eau | qsp 100 % |

Les émulsions sont diposées dans deux compartiments différents et sont mises en contact au moment de l'application.

### Exemple 3 : Crème solaire autobronzante

A. Emulsion contenant l'ester de dihydroxyacétone :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |
| Palmitate de dihydroxyacétone | 14,6 % |

| *Phase aqueuse :* | |
|---|---|
| Phénoxyéthanol (conservateur) | 0,5 % |
| Eau | qsp 100 % |

B. Emulsion contenant de lysine :

| *Phase huileuse :* | |
|---|---|
| Steareth-2 (tensioactif) | 3 % |
| Steareth-21 (tensioactif) | 2 % |
| PPG-15 stearyl éther (tensioactif) | 12 % |
| Alcool cétylique | 3,5 % |
| Vaseline | 5 % |

| *Phase aqueuse :* | |
|---|---|
| Glycérol | 6 % |
| Phénoxyéthanol (conservateur) | 0,5 % |
| Lysine | 5 % |
| Eau | qsp 100 % |

Les émulsions A et B sont disposées dans deux compartiments séparés et mélangées au moment de l'application sur la peau.

Le produit obtenu donne, après application sur la peau, une coloration progressivement bronzée à la peau.

### Exemple 4 : Crème solaire autobronzante

A. Emulsion contenant l'ester de dihydroxyacétone :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |
| Laurate de dihydroxyacétone | 10 % |

| *Phase aqueuse :* | |
|---|---|
| Phénoxyéthanol (conservateur) | 0,5 % |
| Eau | qsp 100 % |

B. Emulsion contenant de lysine :

| *Phase huileuse :* | |
|---|---|
| Steareth-2 (tensioactif) | 3 % |
| Steareth-21 (tensioactif) | 2 % |
| PPG-15 stearyl éther (tensioactif) | 12 % |
| Alcool cétylique | 3,5 % |
| Vaseline | 5 % |

| *Phase aqueuse :* | |
|---|---|
| Glycérol | 6 % |
| Phénoxyéthanol (conservateur) | 0,5 % |
| Lysine | 5 % |
| Eau | qsp 100 % |

Les émulsions sont diposées dans deux compartiments différents et sont mises en contact au moment de l'application.

## Revendications

1. Composition cosmétique ou dermatologique, comprenant, dans un véhicule cosmétiquement ou dermatologiquement acceptable, au moins un dérivé estérifié de la dihydroxyacétone répondant à la formule générale : 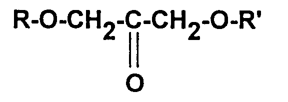 dans laquelle R et R' représentent un atome d'hydrogène ou un radical acyle éventuellement hydroxylé, ayant de 2 à 25 atomes de carbone, linéaire ou ramifié, ou cyclique, saturé ou insaturé, R et R' étant identiques ou différents, à la condition qu'ils ne soient jamais simultanément un atome d'hydrogène.

2. Composition comprenant au moins un dérivé estérifié de la dihydroxyacétone répondant à la formule générale : 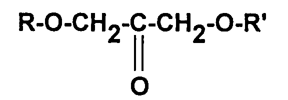 dans laquelle R et R' représentent un atome d'hydrogène ou un radical acyle éventuellement hydroxylé, ayant de 2 à 25 atomes de carbone, linéaire ou ramifié, ou cyclique, saturé ou insaturé, R et R' étant identiques ou différents, à la condition qu'ils ne soient jamais simultanément un atome d'hydrogène,
et au moins un composé capable de couper au moins une liaison ester.

3. Composition selon l'une quelconque des revendications 1 ou 2, dans laquelle le radical acyle est un radical benzoyle ou un radical alkylbenzoyle ou un radical acylbenzoyle ou un radical 2-hydroxy-2-phénylacétyle.

4. Composition selon l'une quelconque des revendications 1 à 3, dans laquelle le radical acyle est hydroxylé.

5. Composition selon l'une quelconque des revendications précédentes, dans laquelle le radical acyle a de 3 à 18 atomes de carbone.

6. Composition selon l'une quelconque des revendications précédentes, dans laquelle le dérivé estérifié est choisi dans le groupe constitué du :
- 1,3-didodécanoate de 2-oxo-propane,
- 1,3-dihéxadécanoate de 2-oxo-propane,
- 3-hexadécanoate de 2-oxo-propanol.

7. Composition selon l'une quelconque des revendications précédentes, dans laquelle le dérivé estérifié est à une concentration allant de 0,1% et 20% en poids par rapport au poids total de la composition.

8. Composition selon l'une quelconque des revendications précédentes dans laquelle le dérivé estérifié est à une concentration allant de 0,5% et 10% en poids par rapport au poids total de la composition.

9. Composition selon l'une quelconque des revendications 2 à 8 dans laquelle le composé capable de couper au moins une liaison ester est un composé nucléophile.

10. Composition selon l'une quelconque des revendications 2 à 9, dans laquelle le composé capable de couper au moins une liaison ester est une amine, ou un anion, ou un enzyme.

11. Composition selon l'une quelconque des revendications 2 à 10, dans laquelle le composé capable de couper au moins une liaison ester est une amine hydroxylée, un anion carboxylate ou une hydrolase.

12. Composition selon l'une quelconque des revendications 2 à 11, dans laquelle le composé capable de couper au moins une liaison ester est choisi parmi : la 3-amino-1,2-propanediol, la 2-amino-2-méthyl-1,3-propanediol, la 2-amino-2-méthyl-propanol, la 2-amino-2-hydroxyméthyl-1,3-propanediol, la glucamine, la n-méthyl-glucamine, une lysine, une arginine, une histidine.

13. Composition selon l'une quelconque des revendications 2 à 12, dans laquelle le composé capable de couper au moins une liaison ester est une lipase, une estérase ou une protéase.

14. Composition selon l'une quelconque des revendications 2 à 13, dans laquelle le composé capable de couper au moins une liaison ester est une lipase.

15. Composition selon l'une quelconque des revendications 2 à 14, dans laquelle le composé capable de couper au moins une liaison ester est à une concentration allant de 0,1% et 30% en poids par rapport au poids total de la composition.

16. Composition selon l'une quelconque des revendications 2 à 15, dans laquelle le composé capable de couper au moins une liaison ester est à une concentration allant de 0,5% et 15% en poids par rapport au poids total de la composition.

17. Composition selon l'une quelconque des revendications 2 à 16, dans laquelle le dérivé estérifié et le composé capable de couper au moins une liaison ester sont conditionnés de sorte à ne pas être au contact l'un de l'autre.

18. Composition selon la revendication 17, dans laquelle le dérivé estérifié et le composé capable de-couper au moins une liaison ester sont contenues dans un seul conditionnement à deux compartiments.

19. Composition selon la revendication 18, dans laquelle l'un au moins du dérivé estérifié ou du composé capable de couper au moins une liaison est encapsulé.

20. Composition selon l'une quelconque des revendications précédentes, caractérisée en ce qu'elle consiste en une composition pour colorer artificiellement la peau.

21. Composition selon l'une quelconque des revendications précédentes, caractérisée en ce qu'elle contient au moins un additif utilisé en cosmétique pour augmenter l'efficacité de la dihydroxyacétone issue de l'hydrolyse du dérivé.

22. Utilisation d'une composition selon l'une quelconque des revendications 1 à 21, pour colorer la peau.

23. Procédé de coloration de la peau consistant à appliquer sur celle-ci une composition selon l'une quelconque des revendications 1 à 21.

## Claims

1. Cosmetic or dermatological composition comprising, in a cosmetically or dermatologically acceptable vehicle, at least one esterified dihydroxyacetone derivative corresponding to the general formula: 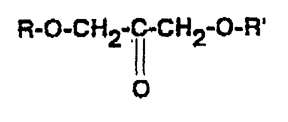 in which R and R' represent a hydrogen atom or a saturated or unsaturated, linear, branched or cyclic, optionally hydroxylated acyl radical having from 2 to 25 carbon atoms, it being possible for R and R' to be identical or different on condition that they are never simultaneously a hydrogen atom.

2. Composition comprising at least one esterified dihydroxyacetone derivative corresponding to the general formula: 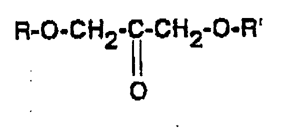 in which R and R' represent a hydrogen atom or a saturated or unsaturated, linear, branched or cyclic, optionally hydroxylated acyl radical having from 2 to 25 carbon atoms, it being possible for R and R' to be identical or different on condition that they are never simultaneously a hydrogen atom,
and at least one compound capable of cleaving at least one ester bond.

3. Composition according to either of Claims 1 and 2, in which the acyl radical is a benzoyl radical or an alkylbenzoyl radical or an acylbenzoyl radical or a 2-hydroxy-2-phenylacetyl radical.

4. Composition according to any one of Claims 1 to 3, in which the acyl radical is hydroxylated.

5. Composition according to any one of the preceding claims, in which the acyl radical has from 3 to 18 carbon atoms.

6. Composition according to any one of the preceding claims, in which the esterified derivative is chosen from the group consisting of:
- 2-oxopropyl 1,3-didodecanoate,
- 2-oxopropyl 1,3-dihexadecanoate,
- 2-oxopropyl 3-hexadecanoate.

7. Composition according to any one of the preceding claims, in which the esterified derivative is at a concentration ranging from 0.1 % to 20 % by weight relative to the total weight of the composition.

8. Composition according to any one of the preceding claims, in which the esterified derivative is at a concentration ranging from 0.5 % to 10 % by weight relative to the total weight of the composition.

9. Composition according to any one of Claims 2 to 8, in which the compound capable of cleaving at least one ester bond is a nucleophilic compound.

10. Composition according to any one of Claims 2 to 9, in which the compound capable of cleaving at least one ester bond is an amine, or an anion, or an enzyme.

11. Composition according to any one of Claims 2 to 10, in which the compound capable of cleaving at least one ester bond is a hydroxylated amine, a carboxylate anion or a hydrolase.

12. Composition according to any one of Claims 2 to 11, in which the compound capable of cleaving at least one eater bond is chosen from: 3-amino-1,2-propanediol, 2-amino-2-methyl-1,3-propanediol, 2-amino-2-methyl-propanol, 2-amino-2-hydroxymethyl-1,3-propanediol, glucamine, N-methylglucamine, a lysine, an arginine, a histidine.

13. Composition according to any one of Claims 2 to 12, in which the compound capable of cleaving at least one ester bond is a lipase, an esterase or a protease.

14. Composition according to any one of Claims 2 to 13, in which the compound capable of cleaving at least one ester bond is a lipase.

15. Composition according to any one of Claims 2 to 14, in which the compound capable of cleaving at least one ester bond is at a concentration ranging from 0.1 % to 30 % by weight relative to the total weight of the composition.

16. Composition according to any one of Claims 2 to 15, in which the compound capable of cleaving at least one ester bond is at a concentration ranging from 0.5 % to 15 % by weight relative to the total weight of the composition.

17. Composition according to any one of Claims 2 to 16, in which the esterified derivative and the compound capable of cleaving at least one ester bond are packaged so as not to be in contact with each other.

18. Composition according to Claim 17, in which the esterified derivative and the compound capable of cleaving at least one ester bond are contained in a single packaging with two compartments.

19. Composition according to Claim 18, in which at least one from among the esterified derivative and the compound capable of cleaving at least one bond is encapsulated.

20. Composition according to any one of the preceding claims, characterized in that it consists of a composition for artificially colouring the skin.

21. Composition according to any one of the preceding claims, characterized in that it contains at least one additive used in cosmetics for increasing the effectiveness of the dihydroxyacetone originating from hydrolysis of the derivative.

22. Use of a composition according to any one of Claims 1 to 21, for colouring the skin.

23. Process for colouring the skin, consisting in applying thereto a composition according to any one of Claims 1 to 21.

## Patentansprüche

1. Kosmetische oder dermatologische Zusammensetzung, die in einem kosmetisch oder dermatologisch akzeptablen Träger mindestens ein Esterderivat von Dihydroxyaceton der allgemeinen Formel 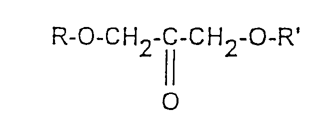 enthält, worin R und R' Wasserstoff oder eine geradkettige, verzweigte oder cyclische, gesättigte oder ungesättigte, gegebenenfalls hydroxyhaltige Acylgruppe mit 2 bis 25 Kohlenstoffatomen bedeuten, wobei R und R' identisch oder voneinander verschieden sind, mit der Maßgabe, daß sie nicht gleichzeitig Wasserstoff bedeuten.

2. Zusammensetzung, die mindestens ein Esterderivat von Dihydroxyaceton der allgemeinen Formel 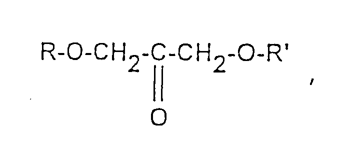 worin R und R' Wasserstoff oder eine geradkettige, verzweigte oder cyclische, gesättigte oder ungesättigte, gegebenenfalls hydroxyhaltige Acylgruppe mit 2 bis 25 Kohlenstoffatomen bedeuten, wobei R und R' identisch oder voneinander verschieden sind, mit der Maßgabe, daß sie nicht gleichzeitig Wasserstoff bedeuten,
und mindestens eine Verbindung enthält, die befähigt ist, mindestens eine Esterbindung zu spalten.

3. Zusammensetzung nach einem der Ansprüche 1 oder 2, worin die Acylgruppe Benzoyl, Alkylbenzoyl, Acylbenzoyl oder 2-Hydroxy-2-phenylacetyl ist.

4. Zusammensetzung nach einem der Ansprüche 1 bis 3, worin die Acylgruppe Hydroxy enthält.

5. Zusammensetzung nach einem der vorhergehenden Ansprüche, wobei die Acylgruppe 3 bis 18 Kohlenstoffatome aufweist.

6. Zusammensetzung nach einem der vorhergehenden Ansprüche, wobei das Esterderivat ausgewählt ist unter:
- 2-Oxo-propan-1,3-didodecanoat,
- 2-Oxo-propan-1,3-dihexadecanoat und
- 2-Oxo-propanol-3-hexadecanoat.

7. Zusammensetzung nach einem der vorhergehenden Ansprüche, wobei das Esterderivat in einer Konzentration im Bereich von 0,1 bis 20 Gew.-%, bezogen auf das Gesamtgewicht der Zusammensetzung, vorliegt.

8. Zusammensetzung nach einem der vorhergehenden Ansprüche, wobei das Esterderivat in einer Konzentration von 0,5 bis 10 Gew.-%, bezogen auf das Gesamtgewicht der Zusammensetzung, vorliegt.

9. Zusammensetzung nach einem der Ansprüche 2 bis 8, wobei die Verbindung, die befähigt ist, mindestens eine Esterbindung zu spalten, eine nucleophile Verbindung ist.

10. Zusammensetzung nach einem der Ansprüche 2 bis 9, wobei die Verbindung, die befähigt ist, mindestens eine Esterbindung zu spalten, ein Amin, ein Anion oder ein Enzym ist.

11. Zusammensetzung nach einem der Ansprüche 2 bis 10, wobei die Verbindung, die befähigt ist, mindestens eine Esterbindung zu spalten, ein hydroxyhaltiges Amin, ein Carboxylatanion oder eine Hydrolase ist.

12. Zusammensetzung nach einem der Ansprüche 2 bis 11, wobei die Verbindung, die befähigt ist, mindestens eine Esterbindung zu spalten, ausgewählt ist unter: 3-Amino-1,2-propandiol, 2-Amino-2-methyl-1,3-propandiol, 2-Amino-2-methyl-propanol, 2-Amino-2-hydroxymethyl-1,3-propandiol, Glucamin, N-Methyl-glucamin, Lysin, Arginin und Histidin.

13. Zusammensetzung nach einem der Ansprüche 2 bis 12, wobei die Verbindung, die befähigt ist, mindestens eine Esterbindung zu spalten, eine Lipase, Esterase oder Protease ist.

14. Zusammensetzung nach einem der Ansprüche 2 bis 13, wobei die Verbindung, die befähigt ist, mindestens eine Esterbindung zu spalten, eine Lipase ist.

15. Zusammensetzung nach einem der Ansprüche 2 bis 14, wobei die Verbindung, die befähigt ist, mindestens eine Esterbindung zu spalten, in einer Konzentration im Bereich von 0,1 bis 30 Gew.-%, bezogen auf das Gesamtgewicht der Zusammensetzung, vorliegt.

16. Zusammensetzung nach einem der Ansprüche 2 bis 15, wobei die Verbindung, die befähigt ist, mindestens eine Esterbindung zu spalten, in einer Konzentration im Bereich von 0,5 bis 15 Gew.-%, bezogen auf das Gesamtgewicht der Zusammensetzung, vorliegt.

17. Zusammensetzung nach einem der Ansprüche 2 bis 16, wobei das Esterderivat und die Verbindung, die befähigt ist, mindestens eine Esterbindung zu spalten, so abgepackt sind, daß sie nicht miteinander in Kontakt stehen.

18. Zusammensetzung nach Anspruch 17, wobei das Esterderivat und die Verbindung, die befähigt ist, mindestens eine Esterbindung zu spalten, in einer einzigen Verpackung mit zwei Abteilungen enthalten sind.

19. Zusammensetzung nach Anspruch 18, wobei das Esterderivat und/oder die Verbindung, die befähigt ist, mindestens eine Bindung zu spalten, eingekapselt sind.

20. Zusammensetzung nach einem der vorhergehenden Ansprüche, dadurch gekennzeichnet, daß sie eine Zusammensetzung zur künstlichen Hautbräunung ist.

21. Zusammensetzung nach einem der vorhergehenden Ansprüche, dadurch gekennzeichnet, daß sie mindestens einen Hilfsstoff enthält, der in der Kosmetik zur Erhöhung der Wirksamkeit des aus der Hydrolyse des Derivats stammenden Dihydroxyacetons verwendet wird.

22. Verwendung einer Zusammensetzung nach einem der Ansprüche 1 bis 21 zur Bräunung der Haut.

23. Verfahren zur Bräunung der Haut, das darin besteht, auf die Haut eine Zusammensetzung nach einem der Ansprüche 1 bis 21 aufzutragen.
